Europäisches Patentamt

European Patent Office

Office européen des brevets

(19)

(11) Publication number: **0 168 289**
**A1**

(12) # EUROPEAN PATENT APPLICATION

(21) Application number: **85401120.2**

(22) Date of filing: **07.06.85**

(51) Int. Cl.⁴: **A 61 M 25/02**

(30) Priority: **07.06.84 US 618018**

(43) Date of publication of application:
**15.01.86 Bulletin 86/3**

(84) Designated Contracting States:
**BE CH DE FR GB IT LI LU NL SE**

(71) Applicant: **ARGON MEDICAL CORP.**
**214 East Corsicana**
**Athens Texas(US)**

(72) Inventor: **Carpenter Rogers, Danny**
**Rte 6, Box 6082**
**Athens Texas(US)**

(72) Inventor: **Nelson Cameron, Donald**
**6323, La Mesa Drive**
**Lago Vista Texas(US)**

(74) Representative: **Descourtieux, Philippe et al,**
**CABINET BEAU de LOMENIE 55 rue d'Amsterdam**
**F-75008 Paris(FR)**

(54) **Flexible collar support.**

(57) The flexible collar support (10) of this invention extends over the tube (12) of a catheter or an introducer sheath to provide resilient collar which supports the tube and prevents it from kinking adjacent to the hub. The flexible collar support (10) comprises a substancially cylindrical body made of a resilient material and having a central bore whose dimensions are substantially the same as the external dimensions of the tube (12), the body being comprised of a first set of disks (14) having substantially wide diameters which are separated from one another by a second set of disks (16) having a substantially narrower diameter.

_Fig. 3_

-1-

## FLEXIBLE COLLAR SUPPORT

The present invention relates to a flexible collar support for use with either a catheter or a catheter introducer sheath to support the tube of the catheter or introducer sheath and prevent the tube from kinking adjacent to the hub. As described herein, reference to the use of the flexible collar support of the present invention with either a catheter or an introducer sheath is intended, where appropriate, to include reference to the use of the invention with the other device. The flexible collar support of the invention is comprised of a flexible collar which fits over the tube of the catheter or introducer sheath, such that the catheter or introducer sheath is slid through a central bore in the flexible collar support. The central bore has dimensions which are essentially the same as the outside dimensions of the catheter with which the flexible collar support is used. The flexible collar support is made up of a series of disks having relatively wide diameters, preferably tapering down from the widest diameter disk, which is adjacent to the hub of the catheter. The first set of disks are connected together by a second set of smaller diameter disks. Axially extending longitudinal stiffeners are preferably used to separate the relatively wide diameter disks from one another. The combination of the wider diameter disks, separated by the narrower diameter disks, together with the axially extending longitudinal stiffeners serve to insure that any

0168289

-2-

bends in the flexible support collar will be relatively smooth. That, together with the fixed internal diameter of the flexible support collar help to prevent kinking of a catheter on which the flexible support collar has been installed by sliding it up the tube to the catheter hub.

In a number of medical applications, catheters and introducer sheaths are used. These typically comprise plastic tubes which are inserted into a vein or artery of a patient. They are used, in the case of catheters, to introduce fluids, typically containing dyes or medication, into a patient. Introducer sheaths are typically used to introduce catheters into a patient's vein or artery. A problem which has existed heretofore has been that such tubes tend to kink if bent. Such kinking generally occurs at the junction of the tube and the end connector or "hub".

Heretofore, a variety of approaches have been used to prevent kinking and cracking adjacent to the hub. For example, the well-known "soda straw" approach of providing wrinkles in the tube adjacent to the hub has been used. While such wrinkles prevent the tube from kinking, internal wrinkles, when exposed to blood flow, may cause blood clotting.

The present invention provides a novel and advantageous structure which overcomes the problems of prior devices.

-3-

In the drawings of an embodiment of the present invention:

FIG. 1 is a side view showing the flexible collar support of the present invention mounted on an introducer sheath;

FIG. 2 is a side view of the flexible collar support of FIG. 1 showing its appearance in a flexed position;

FIG. 3 is a partial cross-sectional view of the flexible collar support of FIG. 1;

FIG. 4 is a cross-sectional view of the flexible collar support of FIG. 1 taken along the line 4-4 of FIG. 3; and

FIG. 5 is a cross-sectional view of the flexible collar support of FIG. 1 taken along the line 5-5 of FIG. 3.

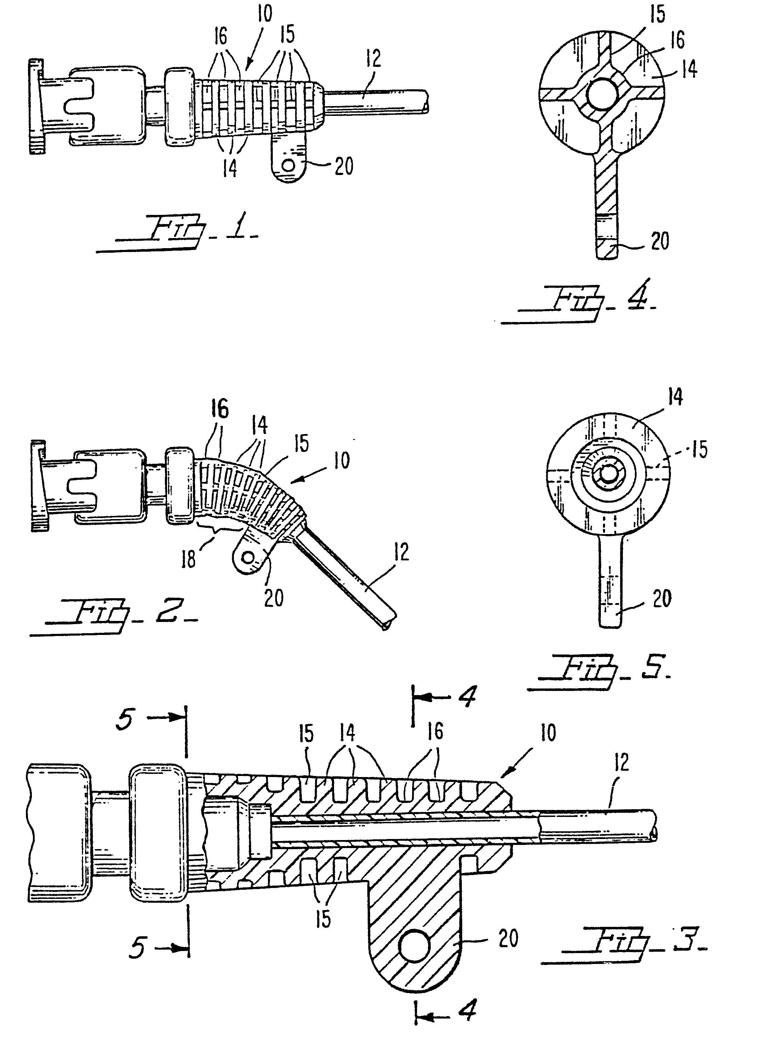

Referring now to FIGS. 1-5, the flexible collar support 10 of the preferred embodiment of the invention is shown. In FIG. 1, the collar support 10 is shown in its normal, relaxed position, while in FIG. 2 the flexible collar support 10 is shown in a flexed position. Sectional views are shown in FIGS. 3-5.

The flexible collar support 10 is comprised of a medium grade soft plastic material, such as soft vinyl or urethane, having a first set of relatively wide diameter annular disks 14 which are attached together by a second set of smaller diameter disks 16 therebetween. Preferably, the wider disks 14 taper from the widest, which is shown on the left in FIG. 1 (adjacent to the hub of

-4-

the introducer sheath 12) to the narrowest diameter disk (which is most remote from the hub of the introducer sheath).

Axially extending supports 15 preferably extend between the wider disks 14 in the longitudinal direction for additional support. These supports 15 bias the disks 14 into their relaxed position, as shown in FIG. 1. The flexible collar support 10 is applied over the shaft an introducer sheath 12 by sliding the shaft through a central bore in the flexible support collar 10. The central bore is dimensioned to conform to the outer dimensions of the shaft of the introducer sheath 12. When the flexible collar support 10 is installed, the introducer sheath 12 can bend, as shown in FIG. 2, with the wider disks 14 closing together over the narrower disks 16, as shown at section 18 of FIG. 2. The provision of the wider disks 14 with the separating narrower disks 16 insures that any bend in the flexible collar support 10 will be made gradually over a portion of the flexible collar support 10. The fixed dimensions of the internal bore of the flexible collar support 10 insures that the introducer sheath 12 will not kink when flexed.

In the preferred embodiment of the invention, the flexible collar support 10 is molded as a collar which is separate from the introducer sheath 12 over which it is applied. The flexible collar support 10 may include a suture tab 20 which includes provision for securing the flexible collar support 10 to a patient, thereby preventing the introducer sheath 12 from shifting position after placement. Typically, the suture tab 20 would only be used on a flexible collar support 10 which was to be applied over an introducer sheath 12, rather than over a catheter (not shown). However, for the purpose of the present invention the flexible collar support 10 would be the same whether it was intended for use with either a catheter or an introducer sheath.

Use of the flexible collar support 10 of the present invention prevents kinking. In addition, it avoids the internal wrinkles of the "soda straw" type of catheters, thereby reducing the risk of blood clotting. The flexible collar support 10 also provides a safety feature when used in conjunction with thin-walled Teflon sheaths in that the external non-kinking feature of the flexible collar support 10 reduces material fatigue when the teflon sheath is in constant patient use. This feature is important, as the thin-walled teflon sheaths which are presently used have been found to crack and leak over a long period of time, and the present invention prevents that from occuring by distributing the bending stress over a greater portion of the tube.

While the preferred embodiment of the invention has been described with reference to use on an introducer sheath, as would be readily understood by those of ordinary skill in the art, the same flexible collar support could be used equally well adjacent to the hub of a catheter. Various changes can be made without departing from the scope of the present invention for particular applications. In particular, the suture tab 20 can

0168289

-6-

be removed or the axially extending supports 15, which are 90° apart in the preferred embodiment of the invention, can be increased or decreased in number. Thus, for particular applications, rather than four axially extending supports, there may be any desired number.

What we claim is:

1.  A flexible collar support for use with a catheter or an introducer sheath comprising a flexible tube having a hub at one end, the flexible collar support comprising a substantially cylindrical body made of a resilient material and having a central bore whose dimensions are substantially the same as the external dimensions of said tube, said body being comprised of a first set of disks having substantially wide diameters which are separated from one another by a second set of disks having a substantially narrower diameter.

2.  The flexible collar support of Claim 1 wherein said resilient material is urethane or vinyl.

3.  The flexible collar support of Claim 1 or 2 wherein the diameter of the disks comprising said first set tapers from the widest disk, adjacent the hub of said catheter, to the narrowest one which is most remote from said hub.

4.  The flexible collar support of Claim 1, 2 or 3 further comprising at least one axially extending longitudinal support made of said resilient material which extends between said disks of said first set along the outer diameter of said disks of said second set.

-8-

5. The flexible collar support of Claim 1, 2, 3 or 4 further comprising a suture tab attached to said flexible collar support whereby a suture can be used to attach said flexible collar support to the body of a patient.

1/1

_Fig_ _1_

_Fig_ _4_

_Fig_ _2_

_Fig_ _5_

_Fig_ _3_

# EUROPEAN SEARCH REPORT

## DOCUMENTS CONSIDERED TO BE RELEVANT

EP 85401120.2

| Category | Citation of document with indication, where appropriate, of relevant passages | Relevant to claim | CLASSIFICATION OF THE APPLICATION (Int Cl 4) |
|---|---|---|---|
| Y | GB - A - 1 181 939 (KABEL U. METALLWERKE) | 1,3 | A 61 M 25/02 |
| A | * Totality; especially fig. 2 * | 2 | |
| | -- | | |
| Y | GB - A - 2 128 481 (V.T. FARRUP) | 1,3 | |
| | * Totality * | | |
| | -- | | |
| A | US - A - 4 203 004 (A.R. COX) | 1-4 | |
| | * Totality; especially column 3, lines 17,18; column 5, lines 25,26 * | | |
| | -- | | |
| A | US - A - 3 950 052 (H.J. WALTER et al.) | 1-4 | |
| | * Totality * | | TECHNICAL FIELDS SEARCHED (Int Cl 4) |
| | -- | | |
| A | US - A - 4 228 825 (W. MORITZ et al.) | 1,2 | A 61 M 25/00 |
| | * Totality * | | F 16 L 11/00 |
| | ---- | | H 01 R 13/00 |
| | | | H 02 G  3/00 |

The present search report has been drawn up for all claims

| Place of search | Date of completion of the search | Examiner |
|---|---|---|
| VIENNA | 07-10-1985 | LUDWIG |